(19) Europäisches Patentamt
European Patent Office
Office européen des brevets

(11) Numéro de publication : **0 388 314 B1**

# (12) FASCICULE DE BREVET EUROPEEN

(45) Date de publication du fascicule du brevet : **11.05.94 Bulletin 94/19**

(51) Int. Cl.[5] : **C07H 15/04,** A61K 31/70, A61K 7/48

(21) Numéro de dépôt : **90400723.4**

(22) Date de dépôt : **16.03.90**

## (54) Esters rétinoiques de l-cladinose, leur procédé de préparation et leur utilisation en médecine humaine ou vétérinaire et en cosmétique.

(30) Priorité : **16.03.89 FR 8903461**

(43) Date de publication de la demande : **19.09.90 Bulletin 90/38**

(45) Mention de la délivrance du brevet : **11.05.94 Bulletin 94/19**

(84) Etats contractants désignés : **AT BE CH DE DK ES FR GB GR IT LI NL SE**

(56) Documents cités :
**EP-A- 0 253 393**
**WO-A-89/00157**
**FR-A- 2 556 348**

(73) Titulaire : **L'OREAL**
**14, Rue Royale**
**F-75008 Paris (FR)**

(72) Inventeur : **Philippe, Michel**
**3, rue de l'Aubépine**
**F-92160 Antony (FR)**
Inventeur : **Sebag, Henri**
**26, rue Erlanger**
**F-75016 Paris (FR)**

(74) Mandataire : **Stalla-Bourdillon, Bernard et al**
**CABINET NONY & CIE 29, rue Cambacérès**
**F-75008 Paris (FR)**

EP 0 388 314 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux esters rétinoïques de L-cladinose, leur procédé de préparation et leur utilisation en médecine humaine et vétérinaire et en cosmétique.

Ces nouveaux esters rétinoïques de L-cladinose trouvent tout particulièrement une application dans le traitement topique et systémique des affections dermatologiques liées à un désordre de la kératinisation (différenciation - prolifération) et d'affections dermatologiques, ou autres, à composantes inflammatoires et/ou immunoallergiques et dans les maladies de dégénérescence du tissu conjonctif, et peuvent présenter une activité anti-tumorale. En outre ces dérivés peuvent être utilisés dans le traitement de l'atopie, qu'elle soit cutanée ou respiratoire et du psoriasis rhumatoïde.

Ils peuvent aussi présenter un intérêt contre le vieillissement de la peau.

Ils trouvent également une application dans le domaine ophtalmologique, notamment dans le traitement des cornéopathies.

Il a déjà été proposé dans le brevet français n°84.18617 (2.556.348) de nouveaux rétinoïdes qui sont des esters ou amides de l'acide étrétinique et d'un sucre, dans le traitement des néoplasies, du psoriasis et de l'acné, ceux-ci répondant à la formule suivante:

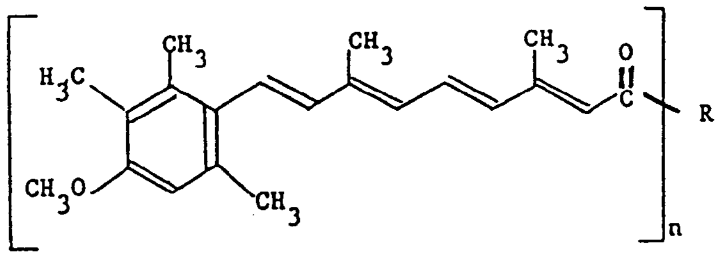

dans laquelle :

R représente le reste d'un sucre relié par une liaison de type ester ou le reste d'un amino sucre relié par une liaison de type amide, ou de dérivés de tels sucres et n est égal à 1 ou 2.

Selon ce brevet, le reste de sucre dérive de préférence du glucose, du maltose, du tréhalose ou du ribose ou encore d'un dérivé de ces sucres.

Après d'importantes études on a constaté de façon tout à fait surprenante et inattendue qu'en utilisant, comme sucre, du L-cladinose, constituant des érythromycines A et B, pour la formation d'esters non seulement de l'acide étrétinique mais également des acides rétinoïques (all trans) et (13 cis), il était possible de remédier aux inconvénients de ces acides à savoir leur toxicité et notamment leur caractère tératogène.

Les études comparatives réalisées ont par ailleurs permis de mettre en évidence que ces nouvelles propriétés étaient dues essentiellement à la nature du sucre utilisé pour l'estérification à savoir le L-cladinose. En effet les esters correspondant de glucose par exemple ne permettent pas la même détoxification notamment de la chaîne rétinoïque all trans.

La présente invention a pour objet, à titre de produits industriels nouveaux, des esters rétinoïques de L-cladinose, ceux-ci pouvant être représentés par la formule générale suivante:

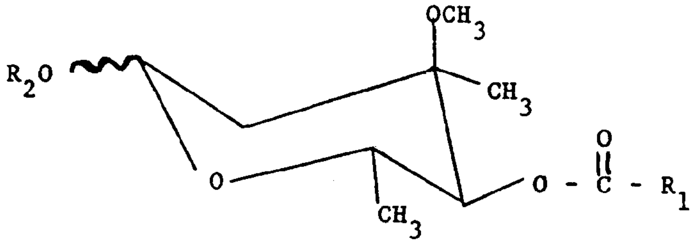

(I)

dans laquelle:

le radical

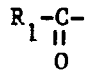

représente soit le radical rétinoyle (all trans) ou (13 cis) soit le radical étrétinoyle, et

$R_2$ représente un radical alkyle, linéaire ou ramifié, ayant de 1 à 24 atomes de carbone,

et les anomères $\alpha$ et $\beta$ et leurs mélanges.

Parmi les radicaux alkyles, linéaires ou ramifiés ayant de 1 à 24 atomes de carbone, on peut notamment mentionner les radicaux méthyle, éthyle, propyle, isopropyle, butyle, tert-butyle, octyle, nonyle, décyle, undécyle, dodécyle, tétradécyle, hexadécyle, octadécyle et décyl-2 tétradécyle.

Lorsque les esters rétinoïques de L-cladinose dérivent de l'acide étrétinique, ceux-ci peuvent être représentés par la formule générale suivante:

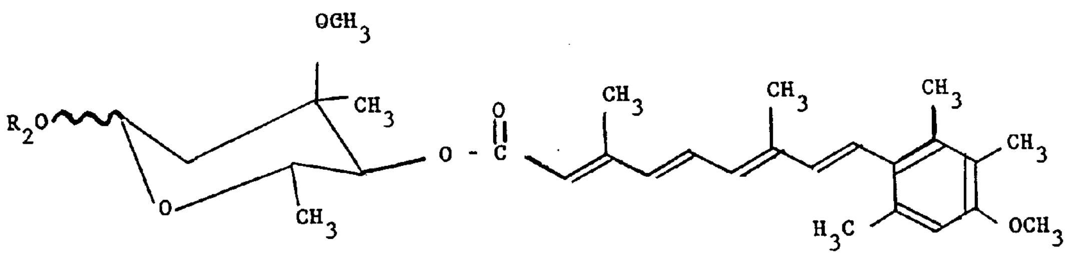

(II)

dans laquelle:

$R_2$ a la même signification que celle donnée ci-dessus pour la formule (I).

Lorsque les esters rétinoïques de L-cladinose dérivent de l'acide rétinoïque (all trans) ou (13 cis), ceux-ci peuvent être représentés par la formule générale suivante:

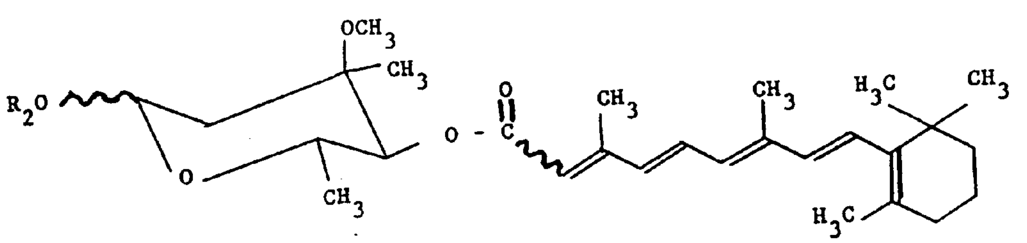

(III)

dans laquelle:

le radical $R_2$ a la même signification que celle donnée ci-dessus pour la formule générale (I).

Parmi les esters rétinoïques de L-cladinose selon l'invention on peut notamment mentionner les suivants:

1) O-rétinoyl (all trans)-4-O-méthyl-1-$\alpha$,$\beta$-L-cladinose,

2) O-rétinoyl (13 cis)-4-O-méthyl-1-$\alpha$,$\beta$-L-cladinose,

3) O-étrétinoyl (all trans)-4-O-méthyl-1-$\alpha$,$\beta$-L-cladinose,

4) O-rétinoyl (all trans)-4-O-décyl-2'-tétradécyl-1-$\alpha$,$\beta$-L-cladinose.

La présente invention a également pour objet le procédé de préparation des esters rétinoïques de L-cladinose tels que définis ci-dessus.

Différents procédés d'estérification du L-cladinose, en position 4, peuvent être utilisés mais de préférence celle-ci est réalisée en milieu solvant organique anhydre comme le tétrahydrofuranne, seul ou en mélange avec un autre solvant organique tel que la pyridine ou le N,N-diméthylformamide, en faisant réagir un excès d'anhydride mixte, soit de l'acide étrétinique, soit de l'acide rétinoïque (all trans) ou (13 cis), préparé in situ, par exemple à partir de chloroformiate d'éthyle et de l'acide choisi, sur un monoéther en position (1) du L-cladinose.

D'autres méthodes d'estérification peuvent être employées, notamment la méthode utilisant les imidazolides des acides choisis, dans un solvant anhydre comme la pyridine ou le N,N-diméthylformamide, en présence d'une base comme le tert-butanolate de potassium ou l'imidazolidure de sodium, mais ces méthodes donnent de manière générale des rendements plus faibles.

Les monoéthers en position 1 du L-cladinose sont obtenus par les méthodes conventionnelles d'étherification en faisant réagir sur le L-cladinose, l'alcool choisi ($R_2$ OH) en présence d'un acide minéral tel que l'acide sulfurique ou chlorhydrique ou d'un acide organique tel que l'acide paratoluène sulfonique, éventuellement dans un solvant organique tel que le N,N-diméthylformamide à une température d'environ 80°C.

Les composés selon l'invention conviennent tout particulièrement dans les domaines suivants:

1) pour traiter des affections dermatologiques liées à un désordre de la kératinisation portant sur la différenciation et sur la prolifération notamment pour traiter les acnés vulgaires, comédoniennes, polymorphes, les acnés nodulo kystiques, conglobata, les acnés séniles, les acnés secondaires telles que l'acné solaire, médicamenteuse, professionnelle;

2) pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen;

3) pour traiter d'autres affections dermatologiques liées à un trouble de la kératinisation avec une composante inflammatoire et/ou immunoallergique et, notamment, toutes les formes de psoriasis qu'il soit cutané, muqueux ou unguéal, et même le rhumatisme psoriasique ou encore l'atopie cutanée, telle que l'eczéma ou l'atopie respiratoire, ces composés peuvent également être utilisés dans certaines affections inflammatoires ne présentant pas de trouble de la kératinisation;

4) pour traiter toutes les proliférations dermiques ou épidermiques qu'elles soient bénignes ou malignes, qu'elles soient d'origine virale telle que verrues vulgaires, les verrues planes et l'épidermodysplasie verruciforme, les proliférations pouvant également être induites par les ultra-violets notamment dans le cadre des épithélioma baso et spino cellulaires;

5) pour traiter d'autres désordres dermatologiques tels que les dermatoses bulleuses et les maladies du collagène;

6) pour traiter certains troubles ophtalmologiques, notamment les cornéopathies;

7) pour lutter contre le vieillissement de la peau, qu'il soit photo-induit ou non;

8) pour prévenir ou guérir les stigmates de l'atrophie épidermique et/ou dermique induite par les corticostéroïdes locaux ou systémiques, ou toute autre forme d'atrophie cutanée.

La présente invention a donc pour objet l'utilisation des esters rétinoïques de L-cladinose tels que définis ci-dessus en tant qu'agents actifs pour des compositions à usage thérapeutique.

Les compositions thérapeutiques destinées notamment au traitement des affections ci-dessus mentionnées, contiennent, dans un support pharmaceutique acceptable, au moins un ester rétinoïque de L-cladinose tel que défini ci-dessus par la formule générale (I).

Ces esters rétinoïques de L-cladinose sont généralement administrés à une dose journalière d'environ 0,01mg/kg à 50mg/kg de poids corporel.

Comme support des compositions on peut utiliser tout support conventionnel, le composé actif se trouvant soit à l'état dissous soit à l'état dispersé dans le véhicule.

L'administration peut être effectuée par voie entérale, parentérale, topique ou oculaire. Par voie entérale les compositions thérapeutiques peuvent se présenter sous forme de comprimés, de gelules, de dragées, de sirops, de suspensions, de solutions, de poudres, de granulés, d'émulsions. Par voie parentérale, les compositions peuvent se présenter sous forme de solutions ou suspensions pour perfusion ou pour injection.

Par voie topique, les compositions thérapeutiques à base des esters rétinoïques de L-cladinose selon l'invention se présentent sous forme d'onguents, de teintures, de crèmes, de pommades, de poudres, de timbres, de tampons imbibés, de solutions, de lotions, de gels, de sprays ou encore de suspensions.

Par voie oculaire, ce sont principalement des collyres.

Ces compositions thérapeutiques contiennent au moins un ester rétinoïque de L-cladinose tel que défini ci-dessus à une concentration de préférence comprise entre 0,001 et 5% par rapport au poids total de la composition.

Les esters rétinoïques de L-cladinose de formule générale (I) trouvent également une application dans le domaine cosmétique en particulier dans l'hygiène corporelle et capillaire et notamment pour le traitement des peaux à tendance acnéïque, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également des compositions cosmétiques contenant dans un support cosmétique acceptable, au moins un ester rétinoïque de L-cladinose de formule générale (I), cette composition se présentant notamment sous forme d'une lotion, d'un gel, d'un savon, d'un shampooing, d'un stick, d'un spray ou d'une mousse aérosols.

La concentration en ester rétinoïque de L-cladinose de formule générale (I) dans les compositions cosmétiques est généralement comprise entre 0,0001 et 5% en poids et de préférence entre 0,001 et 3% en poids.

Les compositions thérapeutiques et cosmétiques selon l'invention peuvent en outre contenir des additifs

inertes ou même pharmacodynamiquement ou cosmétiquement actifs et notamment: des agents hydratants comme la thiamorpholinone et ses dérivés ou l'urée; des agents anti-séborrhéïques ou anti-acnéiques, tels que la S-carboxyméthylcystéine, la S-benzyl-cystéamine et leurs sels et leurs dérivés, la thioxolone ou le peroxyde de benzoyle, des antibiotiques comme l'érythromycine et ses esters, la néomycine, les tétracyclines ou les polyméthylène-4,5 isothiazolinones-3; des agents favorisant la repousse des cheveux, comme le "Minoxidil" (2,4-diamino-6-pipéridino-pyrimidine-3-oxyde) et ses dérivés, le Diazoxide (7-chloro-3-méthyl-1,2,4-benzothiadiazine-1,1-dioxyde) et le Phénytoïn (5,5-diphényl-imidazolidine-2,4-dione); des agents anti-inflammatoires stéroïdiens et non-stéroïdiens; des caroténoïdes et notamment le β-carotène; des agents anti-psoriasiques tels que l'anthraline et ses dérivés et les acides eicosatétraynoïque-5,8,11,14 et triynoïque-5,8,11 leurs esters et leurs amides.

Les compositions selon l'invention qu'elles soient à usage thérapeutique ou cosmétique peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des antioxydants tels que 1'α-tocophérol, le butylhydroxyanisole ou le butylhydroxytoluène.

On va maintenant donner à titre d'illustration et sans aucun caractère limitatif, plusieurs exemples de préparation des composés actifs de formule (I) selon l'invention ainsi que des exemples de composition les contenant.

## EXEMPLES DE PREPARATION

### EXEMPLE 1

#### Préparation du O-rétinoyl (all trans)-4-O-méthyl-1-α,β-L-cladinose

Dans un ballon, sous atmosphère inerte on dissout 5g (16,6mmoles) d'acide rétinoïque (all trans) dans 35ml de tétrahydrofuranne anhydre. Le mélange réactionnel est refroidi à 0°C puis on verse 3ml (38mmoles) de pyridine anhydre et 1,6ml (16,6mmoles) de chloroformiate d'éthyle; la solution est agitée 5 minutes et on ajoute 2,5g (30mmoles) d'hydrogénocarbonate de sodium puis 1,3g (6,8mmoles) de O-méthyl-1-α,β-L-cladinose préalablement dissous dans 150ml de tétrahydrofuranne. Le mélange réactionnel est alors laissé sous agitation pendant 10 heures en laissant remonter à température ambiante (chromatographie sur couche mince de gel de silice: chlorure de méthylène/méthanol 10%). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant: acétate d'éthyle (3)/hexane (7) pour aboutir à l'isolement de 1,9g (59% de rendement) de O-rétinoyl (all trans)-4-O-méthyl-1-α,β-L-cladinose.

Microanalyse : $C_{29}H_{44}O_5$, $1H_2O$; PM : 490,7

| | C | H |
|---|---|---|
| Calc %: | 70,98 | 9,44 |
| Tr %: | 70,40 | 9,34 |

R.M.N. du $^{13}C$ ($CDCl_3$, réf. interne T.M.S.).

La configuration all trans de la chaîne rétinoïque est donnée par le déplacement chimique du $C'_{14}$ à 117,53 ppm. Les effets γ négatifs en 5 (-2,6ppm pour l'anomère α et -2,1ppm pour l'anomère β ) indiquent la position de l'ester en 4.

### EXEMPLE 2

#### Préparation du O-rétinoyl (13 cis)-4-O-méthyl-1-α,β-L-cladinose

Dans un ballon, sous atmosphère inerte, on dissout 10g (33,2mmoles) d'acide rétinoïque (13 cis) dans 90ml de tétrahydrofuranne anhydre; le mélange réactionnel est refroidi à 0°C puis on verse 7ml de pyridine anhydre et 3,2ml (33,2mmoles) de chloroformiate d'éthyle. La solution est agitée 5 minutes et on ajoute 5g d'hydrogénocarbonate de sodium puis 2,5g (13,1mmoles) de O-méthyl-1α,β-L-cladinose préalablement dissous dans 200ml de tétrahydrofuranne. Le mélange réactionnel est alors laissé sous agitation pendant 10 heures en laissant remonter à température ambiante (chromatographie sur couche mince de gel de silice: chlorure de méthylène/méthanol 10%). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant: acétate d'éthyle (3)/hexane (7) pour aboutir à l'isolement de 4g (64% de rendement) de O-rétinoyl (13 cis)-4-O-méthyl-1-α,β-L-cladinose.

Microanalyse : $C_{29}H_{44}O_5$, 1,5$H_2O$; PM : 499,7

| | C | H |
|---|---|---|
| Calc %: | 69,70 | 9,46 |
| Tr %: | 69,58 | 9,34 |

R.M.N. du $^{13}C$ ($CDCl_3$, réf. interne T.M.S.).

Les effets γ négatifs en 5 (-2,6ppm) et 3 (-2,1ppm), respectivement pour les anomères α et β , montrent que l'ester est en position 4. La configuration 13 cis de la chaîne rétinoïque est donnée par le déplacement chimique du $C'_{14}$ à 115,5ppm.

EXEMPLE 3

Préparation du O-étrétinoyl (all trans)-4-O-méthyl-1-α, β-L-cladinose

Dans un ballon, sous atmosphère inerte, on introduit 716 mg (6,6mmoles) de chloroformiate d'éthyle et 20 ml de tétrahydrofuranne. La solution est agitée et refroidie à - 5 °C.

On ajoute alors goutte à goutte, sans dépasser 0°C une solution contenant 2,2g (6,4mmoles) d'acide étrétinique ,660mg (6,6mmoles) de triéthylamine et 20 ml de tétrahydrofuranne. Le milieu est agité encore 1 heure 30 à température ambiante puis débarrassé des sels de triéthylamine par filtration.

Les filtrats sont alors introduits à température ambiante et sous atmosphère inerte, dans un ballon contenant 370 mg (2,1mmoles) de O-méthyl-1-α,β -L-cladinose et 0,6 ml de pyridine anhydre (6,6mmoles) et sous atmosphère inerte.

Le milieu réactionnel est laissé sous agitation pendant 15 heures.

La réaction est suivie par chromatographie sur couche mince de gel de silice en utilisant l'éluant : acétate d'éthyle (6)-heptane (4).

Le milieu est alors concentré puis repris dans du toluène et purifié par chromatographie sur colonne de gel de silice (HPLC) en utilisant l'éluant : acétate d'éthyle (6)-heptane (4) pour aboutir à l'isolement de 180 mg (17,6% de rendement) de O-étrétinoyl (all trans)-4-O-méthyl-1-α,β-L-cladinose.

Microanalyse :

| | | C | H |
|---|---|---|---|
| Calc % | : | 72,26 | 8,49 |
| Tr. % | : | 72,36 | 8,55 |

R.M.N. du $^{13}C$ ($CDCl_3$, réf. interne T.M.S.) : le spectre est en accord avec la structure proposée.

COMPOSITIONS PHARMACEUTIQUES ET COSMETIQUES

A - Gels pour le traitement topique de l'acné

1. Hydroxypropyl cellulose.................. 1g
Butylhydroxytoluène...................... 0,05g
O-rétinoyl (13 cis)-4-O-méthyl-1-α, β-L-cladinose.............................. 0,1g
Isopropanol q.s.p......................... 100g

2. Hydroxypropyl cellulose.................. 1,5g
Butylhydroxytoluène...................... 0,05g
O-rétinoyl (all trans)-4-O-méthyl-1-α,β-L-cladinose................................ 0,075g
Isopropanol q.s.p......................... 100g

B - Lotion pour le traitement topique de l'acné

Butylhydroxytoluène...................... 0,05g
O-rétinoyl (13 cis)-4-O-méthyl-1-α,β-L-cladinose.............................. 0,7g
Triglycérides d'acides gras en $C_8$-$C_{12}$ q.s.p.................................. 100g

C - Stick pour le traitement de l'acné

Vaseline blanche......................... 52,7g
Huile de vaseline........................ 15g
Paraffine raffinée....................... 32g
O-rétinoyl (all trans)-4-O-méthyl-1-α,β-L-cladinose.............................. 0,3g

**Revendications**

1. Esters rétinoïques de L-cladinose, caractérisés par le fait qu'ils répondent à la formule générale suivante:

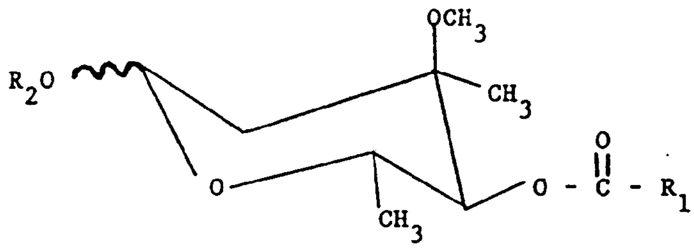

(I)

dans laquelle:

le radical

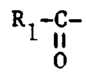

représente soit le radical rétinoyle (all trans) ou (13 cis) soit le radical étrétinoyle, et

$R_2$ représente un radical alkyle, linéaire ou ramifié, ayant de 1 à 24 atomes de carbone,

et les anomères $\alpha$ et $\beta$ et leurs mélanges.

2. Composés selon la revendication 1, caractérisés par le fait que le radical alkyle, linéaire ou ramifié, ayant de 1 à 24 atomes de carbone est le radical méthyle, éthyle, propyle, isopropyle, butyle, tert-butyle, octyle, nonyle, décyle, undécyle, dodécyle, tétradécyle, hexadécyle, octadécyle et décyl-2 tétradécyle.

3. Composés selon la revendication 1, caractérisés par le fait qu'ils dérivent de l'acide étrétinique et répondent à la formule générale suivante:

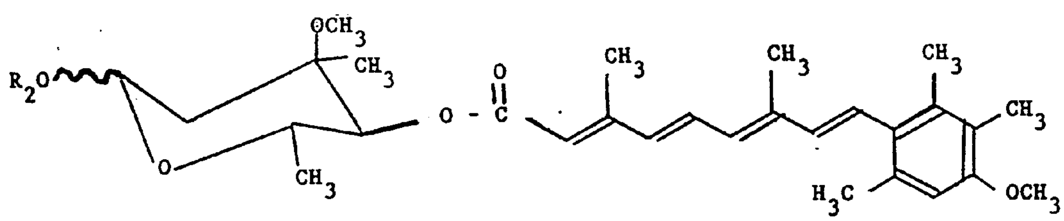

(II)

dans laquelle:

$R_2$ a la même signification que celle donnée à la revendication 1.

4. Composés selon la revendication 1, caractérisés par le fait qu'ils dérivent de l'acide rétinoïque (all trans) ou (13 cis) et répondent à la formule générale suivante:

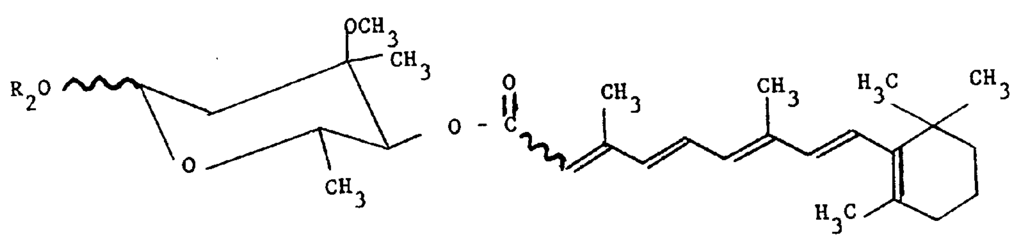

(III)

dans laquelle:

$R_2$ a la même signification que celle donnée à la revendication 1.

5. Composés selon l'une quelconque des revendications 1 à 4, caractérisés par le fait qu'ils sont pris dans le groupe constitué par:

O-rétinoyl (all trans)-4-O-méthyl-1-α,β-L-cladinose,
O-rétinoyl (13 cis)-4-O-méthyl-1-α,β-L-cladinose,
O-étrétinoyl (all trans)-4-O-méthyl-1-α,β-L-cladinose,
O-rétinoyl (all trans)-4-O-décyl-2'-tétradécyl-1-α,β-L-cladinose.

6. Procédé de préparation des composés selon l'une quelconque des revendications 1 à 5, caractérisé par le fait qu'il consiste à faire réagir un excès d'anhydride mixte soit de l'acide étrétinique soit de l'acide rétinoïque (all trans) ou (13 cis), préparé in situ, sur un monoéther de L-cladinose en position (1), la réaction d'estérification étant effectuée en milieu solvant organique.

7. Procédé selon la revendication 6, caractérisé par le fait que le solvant organique est le tétrahydrofuranne, seul ou en mélange avec de la pyridine ou du N,N-diméthylformamide.

8. Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 5.

9. Composition selon la revendication 8, caractérisée par le fait qu'elle se présente sous une forme appropriée pour une application topique et contient de 0,0001 à environ 5% en poids d'un composé de formule (I).

10. Utilisation d'un composé selon l'une quelconque des revendications 1 à 5, pour la préparation d'une composition pharmaceutique destinée au traitement des affections dermatologiques, rhumatismales, respiratoires ainsi qu'ophtalmologiques.

11. Composition cosmétique, caractérisée par le fait qu'elle contient, dans un véhicule cosmétique approprié, au moins un composé de formule (I) selon l'une quelconque des revendications 1 à 5.

12. Composition cosmétique selon la revendication 11, caractérisée par le fait qu'elle contient le composé de formule (I) à une concentration comprise entre 0,0001 et 5% et de préférence entre 0,001 et 3% en poids.

13. Utilisation d'un composé selon l'une quelconque des revendications 1 à 5 pour la préparation d'une composition destinée à lutter contre le vieillissement de la peau.

## Patentansprüche

1. Retinsäureester von L-Cladinose, gekennzeichnet durch folgende allgemeine Formel:

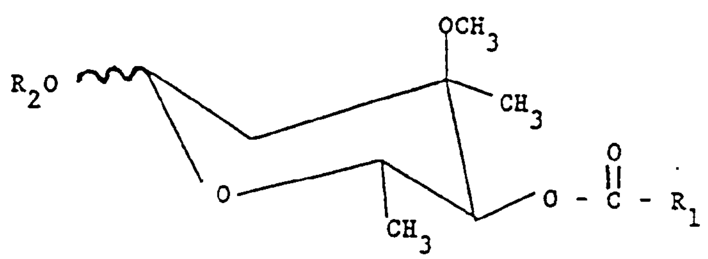

(I)

worin:
das Radikal $R_1$-CO- entweder ein all-trans- oder 13-cis-Retinoylrest oder ein Etretinsäurerest ist, und $R_2$ einen linearen oder verzweigten Alkylrest mit 1 bis 24 Kohlenstoffen darstellt sowie deren α- und β-Anomere und Gemische.

2. Verbindungen gemäß Anspruch 1, dadurch gekennzeichnet, daß der lineare oder verzweigte Alkylrest mit 1 bis 24 Kohlenstoffatomen eine Methyl-, Ethyl-, Propyl-, Isopropyl-, Butyl-, tert.-Butyl-, Octyl-, Nonyl-, Decyl-, Undecyl-, Dodecyl-, Tetradecyl-, Hexadecyl-, Octadecyl- oder Decyl-2-tetradecylgruppe ist.

3. Verbindungen gemäß Anspruch 1, dadurch gekennzeichnet, daß sie Etretmsäurederivate der folgenden

allgemeinen Formel sind:

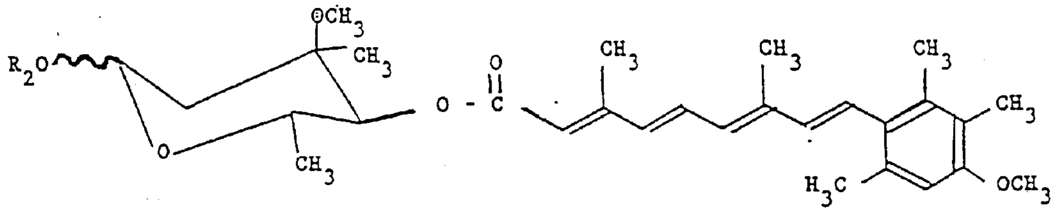

(II)

worin
$R_2$ dieselbe Bedeutung wie in Anspruch 1 hat.

**4.** Verbindungen gemäß Anspruch 1, dadurch gekennzeichnet, daß sie all-trans- oder 13-cis-Retinsäurederivate der folgenden allgemeinen Formel sind:

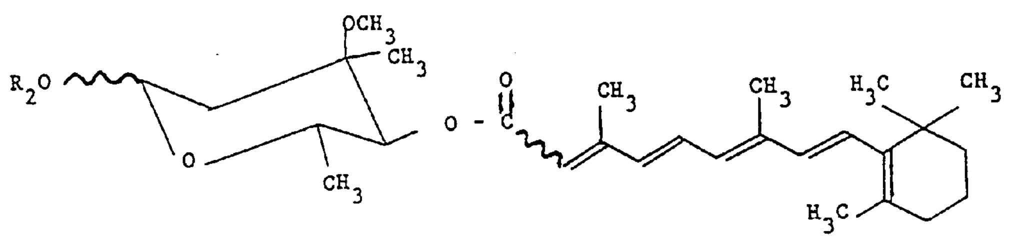

(III)

worin:
$R_2$ die in Anspruch 1 angegebene Bedeutung hat.

**5.** Verbindungen gemäß den Ansprüchen 1 bis 4, dadurch gekennzeichnet, daß sie aus der Gruppe, die aus
4-O-Methyl-α,β-L-cladinose-O-retinat (all-trans),
4-O-Methyl-1-α,β-L-cladinose-O-retinat (13-cis),
4-O-Methyl-1-α,β-L-cladinose-O-etretinat (all-trans), und
4-O-Decyl-2-tetradecyl-α,β-L-cladinose-O-retinat (all-trans)
besteht, ausgewählt sind.

**6.** Verfahren zur Herstellung einer Verbindung gemäß einem der Ansprüche 1 bis 5, dadurch gekennzeichnet, daß man einen Überschuß eines in-situ hergestellten gemischten Anhydrids sowohl der Etretinsäure als auch der all-trans- oder 13-cis-Retinsäure mit einem Monoether in der 1-Stellung der L-Cladinose umsetzt, wobei die Veresterung in einem organischen Lösungsmittel ausgeführt wird.

**7.** Verfahren gemäß Anspruch 6, dadurch gekennzeichnet, daß das Lösungsmittel Tetrahydrofuran allein oder im Gemisch mit Pyridin oder N,N-Dimethylformamid vorliegt.

**8.** Pharmazeutische Zubereitung, dadurch gekennzeichnet, daß sie außer einem zur enteralen, parenteralen, topischen oder ophthalmologischen Applikation geeigneten Träger mindestens eine Verbindung der Formel (I) gemäß einem der Ansprüche 1 bis 5 enthält.

**9.** Zubereitung gemäß Anspruch 8, dadurch gekennzeichnet, daß sie in einer für die topische Applikation geeigneten Form vorliegt und von 0,001 bis ungefähr 5 Gew.-% einer Verbindung gemäß der Formel (I) enthält.

**10.** Verwendung einer Verbindgung gemäß einem der Ansprüche 1 bis 5 zur Herstellung einer pharmazeutischen Zubereitung zur Behandlung dermatologischer oder rheumatischer Erkrankungen oder Erkrankungen der Atemwege sowie der Augen.

**11.** Kosmetische Zubereitung, dadurch gekennzeichnet, daß sie außer einem geeigneten kosmetischen Träger mindestens eine Verbindung der Formel (I) gemäß einem der Ansprüche 1 bis 5 enthält.

**12.** Kosmetische Zubereitung gemäß Anspruch 11, dadurch gekennzeichnet, daß sie eine Verbindung gemäß Anspruch 1 in einer Konzentration von 0,0001 und 5, vorzugsweise von 0,001 bis 3 Gew.-%, enthält.

**13.** Verwendung einer Verbindung gemäß einem der Ansprüche 1 bis 5 zur Herstellung einer Zubereitung zur Bekämpfung der Hautalterung.

**Claims**

**1.** Retinoic esters of L-cladinose, characterized in that they correspond to the following general formula:

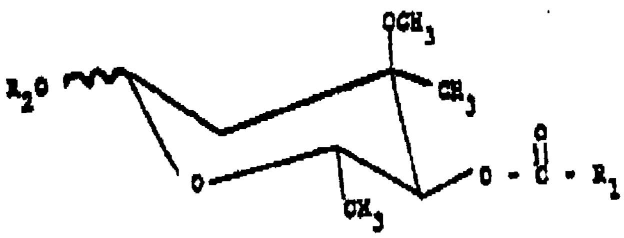

(I)

in which:

the radical

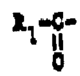

represents either the all-trans- or 13-cis-retinoyl radical or the etretinoyl radical, and

$R_2$ represents a linear or branched alkyl radical having 1 to 24 carbon atoms,

and the $\alpha$ and $\beta$ anomers and their mixtures.

**2.** Compounds according to Claim 1, characterized in that the linear or branched alkyl radical having 1 to 24 carbon atoms is the methyl, ethyl, propyl, isopropyl, butyl, tert-butyl, octyl, nonyl, decyl, undecyl, dodecyl, tetradecyl, hexadecyl, octadecyle or 2-decyltetradecyl radical.

**3.** Compounds according to Claim 1, characterized in that they are derived from etretinic acid and correspond to the following general formula:

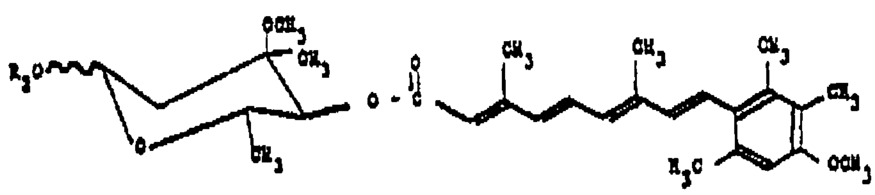

(II)

in which:

$R_2$ has the same meaning as that given in Claim 1.

**4.** Compounds according to Claim 1, characterized in that they are derived from all-trans- or 13-cis-retinoic acid and correspond to the following general formula:

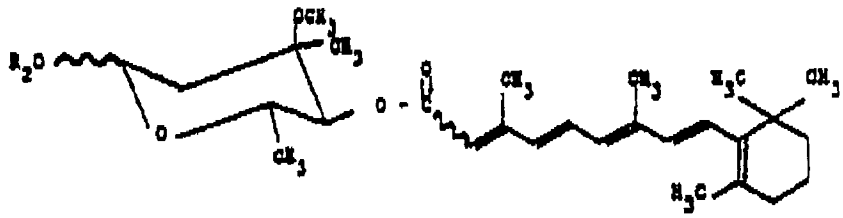

(III)

in which

$R_2$ has the same meaning as that given in Claim 1.

**5.** Compounds according to any one of Claims 1 to 4, characterized in that they are taken from the group consisting of:

4-O-(all-trans-retinoyl)-1-O-methyl-α,β-L-cladinose,
4-O-(13-cis-retinoyl)-1-O-methyl-α-β-L-cladinose,
4-O-(all-trans-etretinoyl)-1-O-methyl-α-β-L-cladinose,
4-O-(all-trans-retinoyl)-2'-O-decyl-1-tetradecyl-α,β-L-cladinose.

**6.** Process for the preparation of the compounds according to any one of Claims 1 to 5, characterized in that it consists in reacting an excess of mixed anhydride either of etretinic acid or of all-trans- or 13-cis-retinoic acid, prepared in situ, with an L-cladinose monoether in position (1), the esterification reaction being performed in an organic solvent medium.

**7.** Process according to Claim 6, characterized in that the organic solvent is tetrahydrofuran, alone or mixed with pyridine or N,N-dimethylformamide.

**8.** Pharmaceutical composition, characterized in that it contains, in a vehicle suitable for administration by the enteral, parenteral, topical or ocular route, at least one compound of formula (I) according to any one of Claims 1 to 5.

**9.** Composition according to Claim 8, characterized in that it is provided in a form suitable for topical application and contains from 0.0001 to approximately 5 % by weight of a compound of formula (I).

**10.** Use of a compound according to any one of Claims 1 to 5, for the preparation of a pharmaceutical composition intended for the treatment of dermatological, rheumatic, respiratory and alos ophthalmological complaints.

**11.** Cosmetic composition, characterized in that it contains, in a suitable cosmetic vehicle, at least one compound of formula (I) according to any one of Claims 1 to 5.

**12.** Cosmetic composition according to Claim 11, characterized in that it contains the compound of formula (I) at a concentration between 0.0001 and 5 %, and preferably between 0.001 and 3 % by weight.

**13.** Use of a compound according to any one of Claims 1 to 5 for the preparation of a composition intended to combat ageing of the skin.